# EUROPEAN PATENT SPECIFICATION

(11) **EP 1 704 847 B1**
(45) Date of publication and mention of the grant of the patent: **12.03.2008**
(21) Application number: 05006342.9
(22) Date of filing: 23.03.2005
(51) Int. Cl.: A61K 8/18

(54) **Cosmetic agents and methods of mirrorizing keratin fibres**
Kosmetische Mittel und Verfahren zur Verspiegelung von Keratinfasern
Produits cosmétiques et procédé de réflection de fibres kératiniques

(43) Date of publication of application: 27.09.2006
(73) Proprietor: Wella Aktiengesellschaft, 64274 Darmstadt (DE)
(72) Inventor: Kripp, Thomas, 64407 Fränkisch-Crumbach (DE); Harpe, Carolin, 64553 Lorsch (DE)

(56) References cited:
- DE-A1- 2 806 603
- FR-A- 1 055 355
- US-A- 3 194 734
- US-A1- 2003 223 944
- PATENT ABSTRACTS OF JAPAN vol. 012, no. 448 (M-768), 24 November 1988 (1988-11-24) & JP 63 179009 A (TANAKA KIKINZOKU KOGYO KK), 23 July 1988 (1988-07-23)

## Description

The present application relates to the use of a combination of a component 1 comprising at least one sulphur-free reducing compound and a component 2 comprising at least one silver salt for achieving a mirrorizing of keratin fibres, and to the use of an agent comprising a sulphur-free reducing compound for freshening or restoring the (silver) lustre of mirrorized hair.

There are a large number of hair colouring methods with, inter alia, also colorations of keratin fibres with mixtures of silver salts and reducing agents being described in the prior art, for example DE 2806603 A1 or US 2003/0223944 A1. According to US 2003/0223944 A1, the keratin fibre is given a metallic shimmer. However, this metallic shimmer does not represent a uniform shiny layer or a silver mirror and is therefore not very satisfactory.

Although there are already products nowadays which supposedly produce a metallic lustre in all imaginable colours on the hair, these are based on metal flakes, lustre pigments of very different types or other highly refractive particles in the combination with polymers or other raw materials which are said to bring about adhesion to the surface of the hair. However, despite their colour-imparting effect, such products are more styling preparations - with all of their system-related disadvantages such as lack of resistance to washing, ready flaking and low abrasion resistance.

There therefore continued to be the object of finding a way of coating the keratin fibres with a uniform, lustrous and resistant silver mirror without metal flakes becoming loose or rubbing off on hand towels, pillows, hats and the like and without a coating flaking off during combing or brushing. This silver mirror on the one hand produces a "new" hair colour, on the other hand, by combining with other suitable colours, the shades known to date can be finished with a mirror lustre or "metallic look".

Surprisingly, it has now been found after numerous experiments that a resistant, bright, penetrable silver lustre (silver mirror) can be produced on keratin fibres if a preparation comprising a sulphur-free reducing compound and an ammoniacal preparation comprising an organic or inorganic silver salt is applied to the keratin fibres at the same time or one after the other.

The present invention therefore provides the use of a combination of a preparation comprising at least one sulphur-free reducing compound which is selected from the group consisting of glyoxylic acid, glyoxal, glycerylaldehyde, maltose, pyruvic acid, erythrulose, fructose, lactose, acetoin, oxalacetic acid, α-ketoglutaric acid, dihydroxyacetone, hydroxymethylglyoxal and 6-aldo-D-fructose and an ammoniacal preparation comprising at least one organic or inorganic silver salt for the mirrorizing of keratin fibres, such as, for example, wool or hair and in particular human hair.

In one particular embodiment of the invention, the mirrorizing can also be combined with suitable colouring methods so that, besides metallic-silver fibres, other metallic-look colours, such as, for example, red metallic or blue metallic, can also be produced.

It is of course not only possible to mirrorize the keratin fibres in their totality, but also to follow fashion trends by mirrorizing individual sections (e.g. hair tresses).

Suitable silver salts are, in particular, silver nitrate, silver chloride, silver sulphate, silver carbonate, silver hydrogensulphate, silver phosphate, silver acetate and silver alums and the corresponding ammonium or thiosulphate complexes of these silver salts.

The amount of silver salts used is, in the ammoniacal silver salt preparation, preferably about 0.5 to 10 per cent by weight, in particular 1 to 4 per cent by weight.

The reducing agent is preferably used in a total amount of from about 0.1 to 15 per cent by weight, in particular 0.5 to 5 per cent by weight.

The pH of the ammoniacal preparation comprising an organic or inorganic silver salt is preferably about 7 to 13, in particular 8 to 12, while the reducing-agent-containing preparation preferably has a pH of from 2 to 12 and in particular 3 to 11.

According to a particularly preferred embodiment of the invention to one or both components (silver salt solution, reducing agent) is also added at least one substance which influences the crystallization and is selected from the group consisting of polyalcohols, amino acids, polymers, organic or inorganic salts, organic bases, salts of organic acids, solvents, sugars, surfactants and emulsifiers or proteins which causes the silver to precipitate out not in microcrystalline form but largely in the form of a mirror layer. Such additives are referred to below as "lustre-imparting agents". Suitable "lustre-imparting agents" are in particular: sorbitol, xylitol, pentaerythritol, glycerol, propylene glycol, serine, histidine, glycine, taurine, polyvinylpyrrolidone, polyvinylpyrrolidone-vinyl acetate copolymers, sodium formate, sodium acetate, sodium oxalate, guanidine carbonate, salts of uric acid, salts of tartaric acid, salts of malonic acid, salts of acetic acid or salts of formic acid, 2-amino-2-methyl-1-propanol, monoethanolamine, sodium thiosulphate, potassium rhodanide, ethanol, isopropanol, acetone, lauryl ether sulphate, PEG-35 castor oil, sodium silicate, cetyltrimethylammonium chloride, ribose, sucrose, trehalose, gelatin and caseins.

The "lustre-imparting agent" is used preferably in a total amount of up to 20 per cent by weight, in particular 0.1 to 10 per cent by weight.

Of course, both the preparation comprising the silver salt and also the reducing-agent-containing preparation - with the exception of oxidizing agents and sulphur-containing reducing agents - can comprise all additives suitable and customary for cosmetic agents: for example perfume oils; dyes; preservatives; solvents, such as water, lower aliphatic alcohols, for example aliphatic alcohols having 1 to 4 carbon atoms, such as ethanol, propanol and isopropanol, or glycols, such as glycerol and 1,2-propylene glycol; wetting agents or emulsifiers from the classes of anionic, cationic, amphoteric or nonionogenic surface-active substances; softeners; petrolatum; silicone oils, paraffin oils and fatty acids, and care substances, such as cationic resins, lanolin derivatives, vitamins, cholesterol, pantothenic acid and betaine. The constituents mentioned are used in the amounts customary for such purposes, for example the wetting agents and emulsifiers in concentrations of from 0.1 to 30 per cent by weight and the care substances in a concentration of from 0.1 to 5 per cent by weight.

The corresponding preparations can be formulated in particular in the form of an aqueous or aqueous-alcoholic preparation, for example in the form of a thickened solution, emulsion, cream or gel, although use in the form of a powder, granules or pellets, which are dissolved prior to use in a suitable aqueous or aqueous-alcoholic preparation, is likewise possible.

The mirrorizing of the keratin fibres, in particular human hair, can be carried out in various ways:
Single-stage method. The reductive component and the silver-salt-containing component are thoroughly mixed together at room temperature, a ratio of 1:1 being particularly preferred. The mixing is preferably carried out directly in the applicator, thereby ensuring that the keratin fibres come into contact with the products as quickly as possible after mixing. An amount of the resulting agent sufficient for the mirrorizing, generally at least 5 ml per gram of the keratin fibres to be mirrorized, is applied evenly to the fibre to be mirrorized. After 0 to 30 minutes, preferably 1 to 10 minutes, the fibre is heated. At the end of the contact time, which is about 1 to 60 minutes, preferably 5 to 20 minutes, the fibre is rinsed with water, optionally washed with a shampoo and dried.
Two-stage method. The reductive component and the silver-salt-containing component are applied to the keratin fibre one after the other, with either the reductive component being applied first and then the silver-salt-containing component, or else the silver-salt-containing component being applied first and then the reductive component. The time between applying the first and second component is about 0 to 60 minutes and in particular 1 to 10 minutes. An amount of the particular component sufficient for the mirrorizing, generally in each case at least 5 ml per gram of the keratin fibre to be mirrorized, is applied evenly to the fibre to be mirrorized. The fibre is then heated. At the end of the contact time, which is about 1 to 60 minutes, preferably 5 to 30 minutes, the fibre is rinsed with water, optionally washed with a shampoo and dried.
Two-stage method with intermediate rinse step. If the first component (reducing agent or silver salt) is used in an increased, at least two-fold concentration, compared with the second component, in the two-stage method described above, the keratin fibre is patted with an absorbent cloth or very briefly rinsed before applying the second component. The time between applying the first and the second component is about 0 to 60 minutes and in particular 1 to 10 minutes. The second component (silver salt or reducing agent) is then applied to the keratin fibres in an amount sufficient for the mirrorizing, generally in each case at least 5 ml per gram of the keratin fibres to be mirrorized and likewise, distributed as evenly as possible. The fibre is then heated. At the end of the contact time, which is about 1 to 60 minutes, preferably 5 to 30 minutes, the fibre is rinsed with water, optionally washed with a shampoo and dried.
Producing a "metallic colour". Before, during or after the single-stage or two-stage mirrorizing described above, the fibre is treated with a suitable, preferably nonoxidative colorant (for example pigments) in the usual way. Preference is given here to hair colorants which colour the cuticle particularly intensively. This gives the shade in question with a metallic lustre.

The treatment temperature on the keratin fibre in the method according to the invention is 20 to 99 °C, particular preference being given to a temperature of at least 50 °C and in particular 50 to 80 °C.

Particularly preferred is the a single-stage method in which the two components are applied at the same time.

It is particularly advantageous if the reduction of the silver ions takes place not only at the surface of the hair, but as deep as possible in the cuticle, and, furthermore, the metallic silver is produced not in microcrystalline form, but in the most continuous layer possible. For this reason, the reaction is preferably controlled such that the silver does not precipitate out suddenly but is somewhat delayed. The silver layer automatically additionally deposited on the surface can be largely removed by thorough shampooing.

The mirrorized keratin fibre should not come into contact with oxidizing agents, such as, for example, hydrogen peroxide since this would destroy the silver mirror. Despite this, if the mirrorized keratin fibre should come into contact with an oxidizing agent, then the original silver lustre can be largely restored by subsequent treatment with a sulphur-free reducing agent which readily penetrates keratin.

The present invention thus further provides agents for freshening or restoring the metallic lustre following oxidizing influences, which are
characterized in that they comprise at least one sulphur-free reducing agent which is selected from the group consisting of glyoxylic acid, glyoxal, glycerylaldehyde, maltose, pyruvic acid, erythrulose, fructose, lactose, acetoin, oxalacetic acid, α-ketoglutaric acid, dihydroxyacetone, hydroxymethylglyoxal and 6-aldo-D-fructose, in a suitable cosmetic base.

The pH of this agent for freshening or restoring the metallic lustre is about 2 to 12 and in particular about 3 to 10.

The agent according to the invention for freshening or restoring the metallic lustre is thoroughly massaged into the keratin fibre (preferably human hair) and left to act with heating to about 20 to 99 °C, preferably about 50 to 80 °C, for about 5 to 30 minutes, preferably 10 to 15 minutes, then rinsed out with water, optionally washed with a shampoo and finally dried.

The examples below are intended to illustrate the subject-matter of the invention without limiting it:

### Examples

### Example 1: Cream-based care silver hair colour

| (Quantitative data in grams) | **Component 1** | **Component 2** |
|---|---|---|
| Glycerol monostearate | 6.0 | 6.0 |
| Lanolin alkoxylate | 2.0 | 2.0 |
| Cetyl alcohol | 2.0 | 2.0 |
| Mixture of lanolin alcohol and paraffin oil (1:1) | 1.0 | 1.0 |
| Tris(oligooxyethyl)alkylammonium phosphate | 1.5 | 1.5 |
| Hydroxyethylcellulose | 1.0 | 1.0 |
| Citric acid | 0.1 | 0.1 |
| Sorbic acid | 0.1 | 0.1 |
| Perfume oil (sulphur-free) | 0.1 | 0.1 |
| Dihydroxyacetone | 1.8 | |
| Ammonia solution, 25% strength aqueous solution | | 4.5 |
| Silver nitrate | | 3.0 |
| Water | ad 100.0 | ad 100.0 |

In each case equal volumes (5 ml per gram of hair to be mirrorized) of component 1 and 2 are thoroughly mixed together at room temperature in an application bottle and applied evenly to the hair to be mirrorized. After a contact time of 5 minutes at room temperature, the hair is heated to 80 °C. After a contact time of 15 minutes, the reaction is largely complete and the mixture can be rinsed out with water. The hair is then thoroughly washed with a shampoo and rinsed again with water. After drying, the hair has a metallic silver lustrous, loose and smooth appearance.

### Example 2: Care silver cream hair colour

| (Quantitative data in grams) | **Component 1** | **Component 2** |
|---|---|---|
| Glycerol monostearate | 6.0 | 6.0 |
| Lanolin alkoxylate | 2.0 | 2.0 |
| Cetyl alcohol | 2.0 | 2.0 |
| Mixture of lanolin alcohol and paraffin oil (1:1) | 1.0 | 1.0 |
| Tris(oligooxyethyl)alkylammonium phosphate | 1.5 | 1.5 |
| Hydroxyethylcellulose | 0.2 | 0.2 |
| Citric acid | 0.1 | 0.1 |
| Taurine | 0.5 | 0.1 |
| Perfume oil (sulphur-free) | 0.1 | 0.1 |
| Erythrulose | 2.5 | |
| Ammonia solution, 25% strength aqueous solution | | 6.5 |
| Silver acetate | | 4.0 |
| Water | ad 100.0 | ad 100.0 |

In each case, at least 5 ml of the component 1 comprising the reducing agent are applied evenly per gram of the hair to be mirrorized. After a contact time of 5 minutes at room temperature, the same volume of the silver-salt-containing component 2 is applied to the section of hair in question and likewise distributed as evenly as possible. It is then heated to 60 to 70 °C. After 25 minutes, the reaction is largely complete and the hair can be rinsed with water. The hair is then also thoroughly washed with a shampoo and again rinsed with water. After drying, the hair has a metallic silver lustrous, loose and smooth appearance.

### Example 3: Mirrorizing cream

| (Quantitative data in grams) | **Component 1** | **Component 2** |
|---|---|---|
| Stearyl alcohol | 8.0 | 8.0 |
| Paraffin oil | 13.0 | 13.0 |
| Wool grease | 6.0 | 6.0 |
| Perfume oil (sulphur-free) | 0.3 | 0.3 |
| Betaine | 0.2 | |
| Glyoxal | 1.5 | |
| Silver nitrate | | 2.5 |
| Ammonia solution, 25% strength aqueous solution | | 5.0 |
| Ethylenediaminotetraacetate disodium salt | 0.2 | 0.2 |
| Water | ad 100.0 | ad 100.0 |

In each case at least 5 ml of the component 1 comprising the reducing agent are applied evenly per gram of the hair to be mirrorized. After a contact time of 5 minutes at room temperature the same volume of the silver-salt-containing component 2 is applied to the section of hair in question and likewise distributed as evenly as possible. It is then heated to 50 to 60 °C. After 40 minutes, the hair is rinsed with water, thoroughly washed with a shampoo and rinsed again with water. After drying, the hair has a metallic silver lustrous, loose and smooth appearance.

### Example 4: Silver cream hair colour

| (Quantitative data in grams) | **Component 1** | **Component 2** |
|---|---|---|
| Stearyl alcohol | 8.0 | 8.0 |
| Paraffin oil | 13.0 | 13.0 |
| Wool grease | 6.0 | 6.0 |
| Perfume oil (sulphur-free) | 0.3 | 0.3 |
| L-histidine | 0.3 | |
| Glycerylaldehyde | 1.0 | |
| Silver carbonate | | 4.0 |
| Ammonia solution, 25% strength aqueous solution | | 5.5 |
| Ethylenediaminotetraacetate disodium salt | 0.2 | 0.2 |
| Water | ad 100.0 | ad 100.0 |

In each case 10 ml of the silver-salt-containing component 2 are applied evenly per gram of the hair to be mirrorized and left to act for 2 minutes at room temperature. The same volume of the component 1 comprising the reducing agent is then applied to the hair and likewise distributed as evenly as possible. The hair is then heated to a temperature of at least 80 °C. After 15 minutes, the hair is rinsed with water, thoroughly washed with a shampoo and rinsed again with water. After drying, the hair has a metallic silver lustrous, loose and smooth appearance.

### Example 5: Silvering solution

| (Quantitative data in grams) | **Component 1** | **Component 2** |
|---|---|---|
| Stearyl alcohol | 0.13 | 0.13 |
| Glyceryl stearate | 0.16 | 0.16 |
| Cetylstearyl alcohol with 20 mol of EO (ceteareth-20) | 0.10 | 0.10 |
| Erythrulose | 1.50 | |
| Dihydroxyacetone | 1.00 | 0.70 |
| Silver acetate | | 4.00 |
| Perfume oil (sulphur-free) | 0.20 | 0.20 |
| Ammonia solution, 25% strength aqueous solution | | 6.00 |
| Ethanol | 5.00 | 5.00 |
| Water | ad 100.00 | ad 100.00 |

In each case 20 ml of the component 2 comprising the silver salt are applied evenly per gram of the hair to be mirrorized and left to act for 5 minutes at room temperature. The hair is then patted with an absorbent cloth. In each case 8 ml of the component 1 comprising the reducing agent per gram of the hair to be mirrorized are then applied to the hair and likewise distributed as evenly as possible. The hair is then heated to 65 to 75 °C. After a contact time of 30 minutes, the hair is rinsed with lukewarm water, thoroughly washed with a shampoo and rinsed again with water. After drying, the hair has a metallic silver lustrous, loose and smooth appearance.

### Example 6: Silver balsam

| (Quantitative data in grams) | **Component 1** | **Component 2** |
|---|---|---|
| Cetyl alcohol | 0.50 | 0.50 |
| Glycerol | 0.30 | 0.30 |
| Cetyltrimethylammonium chloride | 0.25 | 0.25 |
| Styrene/vinylpyrrolidone copolymer | 0.20 | 0.20 |
| Hydroxymethylglyoxal | 1.50 | |
| Silver nitrate | | 2.00 |
| Ammonia solution, 25% strength aqueous solution | | 5.50 |
| Perfume oil (sulphur-free) | 0.20 | 0.20 |
| Ethanol | 4.80 | 4.80 |
| Water | ad 100.00 | ad 100.00 |

The mirrorizing takes place in the manner described in Example 1.
Metallic silver lustrous hair is obtained.

### Example 7: Silver rinse

| (Quantitative data in grams) | **Component 1** | **Component 2** |
|---|---|---|
| Cetylstearyl alcohol | 1.4 | 1.4 |
| Glyceryl stearate | 0.7 | 0.7 |
| Cetylstearyl alcohol with 20 mol of EO (ceteareth-20) | 0.1 | 0.1 |
| Cetyltrimethylammonium chloride | 0.6 | 0.6 |
| 6-aldo-D-fructose | 3.0 | |
| Serine | | 1.5 |
| Silver carbonate | | 1.9 |
| Ammonia solution, 25% strength aqueous solution | | 5.8 |
| Perfume oil (sulphur-free) | 0.4 | 0.4 |
| Water, demineralized | ad 100.0 | ad 100.0 |

The mirrorizing takes place in the manner described in Example 1.
Metallic silver lustrous hair is obtained.

### Example 8: Care silver mirror treatment

| (Quantitative data in grams) | **Component 1** | **Component 2** |
|---|---|---|
| Cetylstearyl alcohol | 5.5 | 5.5 |
| Petrolatum | 1.2 | 1.2 |
| Paraffinum liquidum | 1.0 | 1.0 |
| Dimethylpolysiloxane (Belsil^{®} DM 500) | 0.5 | 0.5 |
| Lanolin alcohol | 0.3 | 0.3 |
| Lanolin | 0.2 | 0.2 |
| Cetyltrimethylammonium chloride | 1.2 | 1.2 |
| Sodium thiosulfate | 0.2 | |
| Dihydroxyacetone | 1.7 | |
| Silver acetate | | 4.0 |
| Ammonia solution, 25% strength aqueous solution | | 6.5 |
| Perfume oil (sulphur-free) | 0.4 | 0.4 |
| Water | ad 100.0 | ad 100.0 |

The mirrorizing takes place in the manner described in Example 2.
Metallic silver lustrous hair is obtained.

### Example 9: Mirrorizing cream

| (Quantitative data in grams) | **Component 1** | **Component 2** |
|---|---|---|
| Cetylstearyl alcohol | 5.5 | 5.5 |
| Petrolatum | 1.2 | 1.2 |
| Paraffinum liquidum | 1.0 | 1.0 |
| Dimethylpolysiloxane (Belsil^{®} DM 500) | 0.5 | 0.5 |
| Lanolin alcohol | 0.3 | 0.3 |
| Lanolin | 0.2 | 0.2 |
| Cetyltrimethylammonium chloride | 1.2 | 1.2 |
| Glyoxylic acid | 2.0 | |
| Pentaerythritol | 0.5 | |
| Silver acetate | | 3.7 |
| Ammonia solution, 25% strength aqueous solution | 2.5 | 5.0 |
| Perfume oil (sulphur-free) | 0.4 | 0.4 |
| Water | ad 100.0 | ad 100.0 |

The mirrorizing takes place in the manner described in Example 1.
Metallic silver lustrous hair is obtained.

### Example 10: Care mirrorizing

| (Quantitative data in grams) | **Component 1** | **Component 2** |
|---|---|---|
| Cetylstearyl alcohol | 6.0 | 6.0 |
| Glycerol | 1.7 | 1.7 |
| Cetyltrimethylammonium chloride | 1.0 | 1.0 |
| Vegetable oil | 1.0 | 1.0 |
| Silicone oil (dimethylpolysiloxane) | 0.5 | 0.5 |
| Perfume oil (sulphur-free) | 0.2 | 0.2 |
| Ethanol | 15.0 | |
| Hydroxymethylglyoxal | 2.0 | |
| Silver carbonate | | 2.5 |
| Ammonia solution, 25% strength aqueous solution | 1.0 | 4.0 |
| Water | ad 100.0 | ad 100.0 |

The mirrorizing takes place in the manner described in Example 5. Metallic silver lustrous hair is obtained.

### Examples 11 to 13: Agents for freshening or restoring the silver mirror

| **Example No.** (quantitative data in grams) | **11** | **12** | **13** |
|---|---|---|---|
| Cetylstearyl alcohol | 6.0 | 6.0 | 6.0 |
| Glycerol | 1.7 | 1.7 | 1.7 |
| Cetyltrimethylammonium chloride | 1.0 | 1.0 | 1.0 |
| Vegetable oil | 1.0 | 1.0 | 1.0 |
| Silicone oil (dimethyl-polysiloxane) | 0.5 | 0.5 | 0.5 |
| Perfume oil (sulphur-free) | 0.2 | 0.2 | 0.2 |
| Dihydroxyacetone | 3.0 | | |
| Hydroxymethylglyoxal | | 3.0 | |
| Glycerylaldehyde | | | 3.0 |
| Glucose | 2.0 | 2.0 | 2.0 |
| Water | ad 100.0 | ad 100.0 | ad 100.0 |

The agent described above for freshening or restoring the silver mirror is in each case thoroughly massaged into the hair and, after a contact time of 10 minutes with heating to at least 50 °C, is rinsed out of the hair with lukewarm water. The hair is then thoroughly washed with a shampoo and rinsed again with water. After drying, the hair has almost completely regained its original silver lustre.

Unless stated otherwise, all of the percentages given in the present application are percentages by weight.

### Example 12: Mirrorizing cream

| (Quantitative data in grams) | **Component 1** | **Component 2** |
|---|---|---|
| Cetylstearyl alcohol | 5.5 | 5.5 |
| Petrolatum | 1.2 | 1.2 |
| Paraffinum liquidum | 1.0 | 1.0 |
| Dimethylpolysiloxane (Belsil^{®} DM 500) | 0.5 | 0.5 |
| Lanolin alcohol | 0.3 | 0.3 |
| Lanolin | 0.2 | 0.2 |
| Cetyltrimethylammonium chloride | 1.2 | 1.2 |
| Glyoxylic acid | 2.0 | |
| Pentaerythritol | 0.5 | |
| Silver acetate | | 3.7 |
| Ammonia solution, 25% strength aqueous solution | 2.5 | 5.0 |
| Perfume oil (sulphur-free) | 0.4 | 0.4 |
| Water | ad 100.0 | ad 100.0 |

The mirrorizing takes place in the manner described in Example 1.
Metallic silver lustrous hair is obtained.

### Example 13: Care mirrorizing

| (Quantitative data in grams) | **Component 1** | **Component 2** |
|---|---|---|
| Cetylstearyl alcohol | 6.0 | 6.0 |
| Glycerol | 1.7 | 1.7 |
| Cetyltrimethylammonium chloride | 1.0 | 1.0 |
| Vegetable oil | 1.0 | 1.0 |
| Silicone oil (dimethylpolysiloxane) | 0.5 | 0.5 |
| Perfume oil (sulphur-free) | 0.2 | 0.2 |
| Ethanol | 15.0 | |
| Hydroxymethylglyoxal | 2.0 | |
| Silver carbonate | | 2.5 |
| Ammonia solution, 25% strength aqueous solution | 1.0 | 4.0 |
| Water | ad 100.0 | ad 100.0 |

The mirrorizing takes place in the manner described in Example 5.
Metallic silver lustrous hair is obtained.

### Example 14: Gel-based mirrorizing mass

| (Quantitative data in grams) | **Component 1** | **Component 2** |
|---|---|---|
| Cellulose ether | 4.0 | 4.0 |
| Hydrogenated castor oil with 45 mol of EO (Cremophor^{®} RH 410) | 0.2 | 2.0 |
| Glycerol 86% | 30.0 | 30.0 |
| Sorbitol | 0.5 | |
| Malondialdehyde tetraethylacetal | 2.5 | |
| Citric acid | 0.3 | |
| Silver nitrate | | 2.9 |
| Ammonia solution, 25% strength aqueous solution | 0.3 | 6.5 |
| Perfume oil (sulphur-free) | 0.1 | 0.1 |
| Water, demineralized | ad 100.0 | ad 100.0 |

The mirrorizing takes place in the manner described in Example 1. Metallic silver lustrous hair is obtained.

### Examples 15 to 20: Agents for freshening or restoring the silver mirror

| **Example No.** (quantitative data in grams) | **15** | **16** | **17** | **18** | **19** | **20** |
|---|---|---|---|---|---|---|
| Cetylstearyl alcohol | 6.0 | 6.0 | 6.0 | 6.0 | 6.0 | 6.0 |
| Glycerol | 1.7 | 1.7 | 1.7 | 1.7 | 1.7 | 1.7 |
| Cetyltrimethylammonium chloride | 1.0 | 1.0 | 1.0 | 1.0 | 1.0 | 1.0 |
| Vegetable oil | 1.0 | 1.0 | 1.0 | 1.0 | 1.0 | 1.0 |
| Silicone oil (dimethyl-polysiloxane) | 0.5 | 0.5 | 0.5 | 0.5 | 0.5 | 0.5 |
| Perfume oil (sulphur-free) | 0.2 | 0.2 | 0.2 | 0.2 | 0.2 | 0.2 |
| Dihydroxyacetone | 3.0 | | | | | |
| Hydroxymethylglyoxal | | 3.0 | | | | |
| Ascorbic acid | | | 3.0 | | | |
| Glutardialdehyde | | | | 3.0 | | |
| Glycerylaldehyde | | | | | 3.0 | |
| Glucose | 2.0 | 2.0 | 2.0 | 2.0 | 2.0 | 2.0 |
| Water | ad 100.0 | ad 100.0 | ad 100.0 | ad 100.0 | ad 100.0 | ad 100.0 |

The agent described above for freshening or restoring the silver mirror is in each case thoroughly massaged into the hair and, after a contact time of 10 minutes with heating to at least 50 °C, is rinsed out of the hair with lukewarm water. The hair is then thoroughly washed with a shampoo and rinsed again with water. After drying, the hair has almost completely regained its original silver lustre.

Unless stated otherwise, all of the percentages given in the present application are percentages by weight.

## Claims

1. Use of a combination of (i) a preparation comprising at least one sulphur-free reducing compound which is selected from the group consisting of glyoxylic acid, glyoxal, glycerylaldehyde, maltose, pyruvic acid, erythrulose, fructose, lactose, acetoin, oxalacetic acid, α-ketoglutaric acid, dihydroxyacetone, hydroxymethylglyoxal and 6-aldo-D-fructose and (ii) an ammoniacal preparation comprising at least one organic or inorganic silver salt for the mirrorizing of keratin fibres.

2. Use according to Claim 1, **characterized in that** the silver salt is selected from the group consisting of silver nitrate, silver chloride, silver sulphate, silver carbonate, silver hydrogensulphate, silver phosphate, silver acetate and silver alums and the corresponding ammonium or thiosulphate complexes of these silver salts.

3. Use according to Claim 1 or 2, **characterized in that** the silver salt in the silver salt preparation is used in a total amount of 0.5 to 10 per cent by weight.

4. Use according to one of Claims 1 to 3, **characterized in that** the reducing agent is present in the reducing-agent-containing preparation in a total amount of from 0.1 to 15 per cent by weight.

5. Use according to one of Claims 1 to 4, **characterized in that** the preparation comprising the silver salt has a pH of from 7 to 13.

6. Use according to one of Claims 1 to 5, **characterized in that** one or both preparations (silver salt solution, reducing agent) comprise at least one substance which influences the crystallization and which is selected from the group consisting of sorbitol, xylitol, pentaerythritol, glycerol, propylene glycol, serine, histidine, glycine, taurine, polyvinylpyrrolidone, polyvinylpyrrolidone-vinyl acetate copolymers, sodium formate, sodium acetate, sodium oxalate, guanidine carbonate, salts of uric acid, salts of tartaric acid, salts of malonic acid, salts of acetic acid, salts of formic acid, 2-amino-2-methyl-1-propanol, monoethanolamine, sodium thiosulphate, potassium rhodanide, ethanol, isopropanol, acetone, lauryl ether sulphate, PEG-35 castor oil, sodium silicate, cetyltrimethylammonium chloride, ribose, sucrose, trehalose, gelatin and caseins.

7. Agent for freshening or restoring the metallic lustre of mirrorized keratin fibres following oxidizing influences, **characterized in that** it comprises in a suitable cosmetic base at least one sulphur-free reducing agent which is selected from the group consisting of glyoxylic acid, glyoxal, glycerylaldehyde, glucose, maltose, pyruvic acid, erythrulose, fructose, oxalacetic acid, α-ketoglutaric acid, ascorbic acid, sodium borohydride, dihydroxyacetone, hydroxymethylglyoxal and 6-aldo-D-fructose.

8. Method of mirrorizing keratin fibres in which a reductive preparation comprising at least one sulphur-free reducing compound which is selected from the group consisting of glyoxylic acid, glyoxal, glycerylaldehyde, maltose, pyruvic acid, erythrulose, fructose, lactose, acetoin, oxalacetic acid, α-ketoglutaric acid, dihydroxyacetone, hydroxymethylglyoxal and 6-aldo-D-fructose and a silver-salt-containing preparation are thoroughly mixed together at room temperature, then are applied evenly to the keratin fibre to be mirrorized and, after a contact time of from 1 to 60 minutes at 20 to 99 °C, the fibre is rinsed with water, optionally washed with a shampoo and finally dried.

9. Method of mirrorizing keratin fibres in which a reductive preparation comprising at least one sulphur-free reducing compound which is selected from the group consisting of glyoxylic acid, glyoxal, glycerylaldehyde, maltose, pyruvic acid, erythrulose, fructose, lactose, acetoin, oxalacetic acid, α-ketoglutaric acid, dihydroxyacetone, hydroxymethylglyoxal and 6-aldo-D-fructose and a silver-salt-containing preparation are applied uniformly in any order one after the other to the keratin fibre to be mirrorized and, after a contact time of from 1 to 60 minutes at 20 to 99 °C, the fibre is rinsed with water, optionally washed with a shampoo and finally dried.

10. Method according to Claim 9, **characterized in that** the initially applied preparation (reducing agent or silver salt) is used in an at least two-fold concentration compared with the second component and before applying the second component the keratin fibres are patted with an absorbent cloth or very briefly rinsed.

11. Method according to Claim 9 or 10, **characterized in that** the time between applying the first preparation and the second preparation is up to 60 minutes.

12. Method according to one of Claims 8 to 11, **characterized in that** the keratin fibre is heated to 50 to 80 °C.

13. Method according to one of Claims 8 to 12, **characterized in that** before, during or after mirrorizing, the keratin fibre is coloured in the usual manner with a suitable colorant.

14. Method according to Claim 13, **characterized in that** a nonoxidative colorant is used.

15. Two-component agent for mirrorizing keratin fibres, **characterized in that** it consists of a preparation comprising at least one sulphur-free reducing compound which is selected from the group consisting of glyoxylic acid, glyoxal, glycerylaldehyde, maltose, pyruvic acid, erythrulose, fructose, lactose, acetoin, oxalacetic acid, α-ketoglutaric acid, dihydroxyacetone, hydroxymethylglyoxal and 6-aldo-D-fructose and an ammoniacal preparation comprising at least one organic or inorganic silver salt.

16. Agent according to Claim 15, **characterized in that** the silver salt is selected from the group consisting of silver nitrate, silver chloride, silver sulphate, silver carbonate, silver hydrogensulphate, silver phosphate, silver acetate and silver alums, and the corresponding ammonium or thiosulphate complexes of these silver salts.

## Patentansprüche

1. Verwendung einer Kombination von (i) einer Zubereitung, umfassend mindestens eine schwefelfreie reduzierende Verbindung, die ausgewählt ist aus der Gruppe, bestehend aus Glyoxylsäure, Glyoxal, Glycerylaldehyd, Maltose, Pyruvinsäure, Erythrulose, Fructose, Lactose, Acetoin, Oxalessigsäure, α-Ketoglutarsäure, Dihydroxyaceton, Hydroxymethylglyoxal und 6-Aldo-D-fructose, und (ii) einer ammoniakalischen Zubereitung, umfassend mindestens ein organisches oder anorganisches Silbersalz zum Verspiegeln von Keratinfasern.

2. Verwendung nach Anspruch 1, **dadurch gekennzeichnet, dass** das Silbersalz ausgewählt ist aus der Gruppe, bestehend aus Silbernitrat, Silberchlorid, Silbersulfat, Silbercarbonat, Silberhydrogensulfat, Silberphosphat, Silberacetat und Silberalaunen und den entsprechenden Ammonium- oder Thiosulfatkomplexen dieser Silbersalze.umfassendddddddddd

3. Verwendung nach Anspruch 1 oder 2, **dadurch gekennzeichnet, dass** das Silbersalz in der Silbersalzzubereitung in einer Gesamtmenge von 0,5 bis 10 Gewichtsprozent verwendet wird.

4. Verwendung nach einem der Ansprüche 1 bis 3, **dadurch gekennzeichnet, dass** das Reduktionsmittel in der reduktionsmittelhaltigen Zubereitung in einer Gesamtmenge von 0,1 bis 15 Gewichtsprozent vorhanden ist.

5. Verwendung nach einem der Ansprüche 1 bis 4, **dadurch gekennzeichnet, dass** die Zubereitung, die das Silbersalz umfasst, einen pH von 7 bis 13 hat.

6. Verwendung nach einem der Ansprüche 1 bis 5, **dadurch gekennzeichnet, dass** eine oder beide Zubereitungen (Silbersalzlösung, Reduktionsmittel) mindestens eine Substanz umfassen, die die Kristallisation beeinflusst und die ausgewählt ist aus der Gruppe, bestehend aus Sorbit, Xylit, Pentaerythrit, Glycerol, Propylenglycol, Serin, Histidin, Glycin, Taurin, Polyvinylpyrrolidon, Polyvinylpyrrolidon-Vinylacetat-Copolymeren, Natriumformiat, Natriumacetat, Natriumoxalat, Guanidincarbonat, Salzen von Harnsäure, Salzen von Weinsäure, Salzen von Malonsäure, Salzen von Essigsäure, Salzen von Methansäure, 2-Amino-2-methyl-1-propanol, Monoethanolamin, Natriumthiosulfat, Kaliumrhodanid, Ethanol, Isopropanol, Aceton, Laurylethersulfat, PEG-35-Rizinusöl, Natriumsilicat, Cetyltrimethylammoniumchlorid, Ribose, Saccharose, Trehalose, Gelatine und Caseinen.

7. Mittel zum Auffrischen oder Wiederherstellen des metallischen Schimmers verspiegelter Keratinfasern nach oxidierenden Einflüssen, **dadurch gekennzeichnet, dass** es in einer geeigneten kosmetischen Basis mindestens ein schwefelfreies Reduktionsmittel umfasst, das ausgewählt ist aus der Gruppe, bestehend aus Glyoxylsäure, Glyoxal, Glycerylaldehyd, Glucose, Maltose, Pyruvinsäure, Erythrulose, Fructose, Oxalessigsäure, α-Ketoglutarsäure, Ascorbinsäure, Natriumborhydrid, Dihydroxyaceton, Hydroxymethylglyoxal und 6-Aldo-D-fructose.

8. Verfahren zum Verspiegeln von Keratinfasern, in dem eine reduzierende Zubereitung, umfassend mindestens eine schwefelfreie reduzierende Verbindung, die ausgewählt aus der Gruppe, bestehend aus Glyoxylsäure, Glyoxal, Glycerylaldehyd, Maltose, Pyruvinsäure, Erythrulose, Fructose, Lactose, Acetoin, Oxalessigsäure, α-Ketoglutarsäure, Dihydroxyaceton, Hydroxymethylglyoxal und 6-Aldo-D-fructose, und eine silbersalzhaltige Zubereitung bei Raumtemperatur gründlich zusammengemischt werden, dann gleichmäßig auf die zu verspiegelnde Keratinfaser aufgetragen werden, und nach einer Kontaktzeit von 1 bis 60 Minuten bei 20 bis 99 °C die Faser mit Wasser gespült, wahlweise mit einem Shampoo gewaschen und schließlich getrocknet wird.

9. Verfahren zum Verspiegeln von Keratinfasern, in dem eine reduzierende Zubereitung, umfassend mindestens eine schwefelfreie reduzierende Verbindung, die ausgewählt ist aus der Gruppe, bestehend aus Glyoxylsäure, Glyoxal, Glycerylaldehyd, Maltose, Pyruvinsäure, Erythrulose, Fructose, Lactose, Acetoin, Oxalessigsäure, α-Ketoglutarsäure, Dihydroxyaceton, Hydroxymethylglyoxal und 6-Aldo-D-fructose, und eine silbersalzhaltige Zubereitung in beliebiger Reihenfolge nacheinander gleichmäßig auf die zu verspiegelnde Keratinfaser aufgetragen werden, und nach einer Kontaktzeit von 1 bis 60 Minuten bei 20 bis 99 °C die Faser mit Wasser gespült, wahlweise mit einem Shampoo gewaschen und schließlich getrocknet wird.

10. Verfahren nach Anspruch 9, **dadurch gekennzeichnet, dass** die zuerst aufgetragene Zubereitung (Reduktionsmittel oder Silbersalz) in einer mindestens zweifachen Konzentration im Vergleich zu dem zweiten Bestandteil aufgetragen wird und vor dem Auftragen des zweiten Bestandteils die Keratinfasern mit einem absorbierenden Tuch abgetupft oder sehr kurz gespült werden.

11. Verfahren nach Anspruch 9 oder 10, **dadurch gekennzeichnet, dass** die Zeit zwischen dem Auftragen der ersten Zubereitung und der zweiten Zubereitung bis zu 60 Minuten beträgt.

12. Verfahren nach einem der Ansprüche 8 bis 11, **dadurch gekennzeichnet, dass** die Keratinfaser auf 50 bis 80 °C erwärmt wird.

13. Verfahren nach einem der Ansprüche 8 bis 12, **dadurch gekennzeichnet, dass** vor, während oder nach der Verspiegelung die Keratinfaser auf gewöhnliche Weise mit einem geeigneten Farbstoff gefärbt wird.

14. Verfahren nach Anspruch 13, **dadurch gekennzeichnet, dass** ein nichtoxidativer Farbstoff verwendet wird.

15. Mittel aus zwei Bestandteilen zum Verspiegeln von Keratinfasern, **dadurch gekennzeichnet, dass** es aus einer Zubereitung besteht, umfassend mindestens eine schwefelfreie reduzierende Verbindung, die ausgewählt ist aus der Gruppe, bestehend aus Glyoxylsäure, Glyoxal, Glycerylaldehyd, Maltose, Pyruvinsäure, Erythrulose, Fructose, Lactose, Acetoin, Oxalessigsäure, α-Ketoglutarsäure, Dihydroxyaceton, Hydroxymethylglyoxal und 6-Aldo-D-fructose, und einer ammoniakalischen Zubereitung, umfassend mindestens ein organisches oder anorganisches Silbersalz.

16. Mittel nach Anspruch 15, **dadurch gekennzeichnet, dass** das Silbersalz ausgewählt ist aus der Gruppe, bestehend aus Silbernitrat, Silberchlorid, Silbersulfat, Silbercarbonat, Silberhydrogensulfat, Silberphosphat, Silberacetat und Silberalaunen und den entsprechenden Ammonium- oder Thiosulfatkomplexen dieser Silbersalze.

## Revendications

1. Utilisation d'une combinaison de (i) une préparation comprenant au moins un composé réducteur exempt de soufre qui est choisi dans le groupe constitué d'acide glyoxylique, glyoxal, glycérylaldéhyde, maltose, acide pyruvique, érythrulose, fructose, lactose, acétoine, acide oxalacétique, acide α-cétoglutarique, dihydroxyacétone, hydroxyméthylglyoxal et 6-aldo-D-fructose et (ii) une préparation ammoniacale comprenant au moins un sel d'argent organique ou inorganique pour donner un effet miroir à des fibres de kératine.

2. Utilisation selon la revendication 1, **caractérisé en ce que** le sel d'argent est choisi dans le groupe constitué de nitrate d'argent, chlorure d'argent, sulfate d'argent, carbonate d'argent, hydrogénosulfate d'argent, phosphate d'argent, acétate d'argent et aluns d'argent, et les complexes ammonium ou thiosulfate correspondants de ces sels d'argent.

3. Utilisation selon la revendication 1 ou 2, **caractérisée en ce que** le sel d'argent dans la préparation de sel d'argent est utilisé en une quantité totale de 0,5 à 10 pour cent en poids.

4. Utilisation selon l'une des revendications 1 à 3, **caractérisée en ce que** l'agent réducteur est présent dans la préparation contenant un agent réducteur en une quantité totale allant de 0,1 à 15 pour cent en poids.

5. Utilisation selon l'une des revendications 1 à 4, **caractérisée en ce que** la préparation comprenant le sel d'argent a un pH allant de 7 à 13.

6. Utilisation selon l'une des revendications 1 à 5, **caractérisée en ce qu'**une ou l'une et l'autre préparations (solution de sel d'argent, agent réducteur) comprennent au moins une substance qui influence la cristallisation et qui est choisie dans le groupe constitué de sorbitol, xylitol, penta-érythritol, glycérol, propylène glycol, sérine, histidine, glycine, taurine, polyvinylpyrrolidone, copolymères polyvinylpyrrolidone-acétate vinylique, formiate de sodium, acétate de sodium, oxalate de sodium, carbonate de guanidine, sels d'acide urique, sels d'acide diacétyltartrique, sels d'acide malonique, sels d'acide acétique, sels d'acide formique, 2-amino-2-méthyl-1-propanol, monoéthanolamine, thiosulfate de sodium, rhodanure de potassium, éthanol, isopropanol, acétone, lauryl éther sulfate, PEG-35 huile de ricin, silicate de sodium, chlorure de cétyltriméthylammonium, ribose, saccharose, tréhalose, gélatine et caséines.

7. Agent pour rafraîchir ou restaurer le lustre métallique de fibres de kératine ayant reçu un effet miroir après des influences oxydantes, **caractérisé en ce qu'**il comprend dans une base cosmétique appropriée au moins un agent réducteur exempt de soufre qui est choisi dans le groupe constitué d'acide glyoxylique, glyoxal, glycérylaldéhyde, glucose, maltose, acide pyruvique, érythrulose, fructose, acide oxalacétique, acide α-cétoglutarique, acide ascorbique, borhydrure de sodium, dihydroxyacétone, hydroxyméthylglyoxal et 6-aldo-D-fructose.

8. Procédé pour donner un effet miroir à des fibres de kératine dans lequel une préparation réductrice qui est choisie dans le groupe constitué d'acide glyoxylique, glyoxal, glycérylaldéhyde, maltose, acide pyruvique, érythrulose, fructose, lactose, acétoine, acide oxalacétique, acide α-cétoglutarique, dihydroxyacétone, hydroxyméthylglyoxal et 6-aldo-D-fructose et une préparation contenant un sel d'argent sont énergiquement mélangées ensemble à la température ambiante, puis appliquées uniformément sur la fibre de kératine à laquelle on veut donner un effet miroir et, après un temps de contact allant de 1 à 60 minutes à 20 à 99 °C, la fibre est rincée avec de l'eau, facultativement lavée avec un shampooing et enfin séchée.

9. Procédé pour donner un effet miroir à des fibres de kératine dans lequel une préparation réductrice comprenant au moins un composé réducteur exempt de soufre qui est choisi dans le groupe constitué d'acide glyoxylique, glyoxal, glycérylaldéhyde, maltose, acide pyruvique, érythrulose, fructose, lactose, acétoine, acide oxalacétique, acide α-cétoglutarique, dihydroxyacétone, hydroxyméthylglyoxal et 6-aldo-D-fructose et une préparation contenant un sel d'argent sont appliquées uniformément dans n'importe quel ordre l'une après l'autre sur la fibre de kératine destinée à être miroirisée et, après un temps de contact allant de 1 à 60 minutes à 20 à 99 °C, la fibre est rincée avec de l'eau, facultativement lavée avec un shampooing et enfin séchée.

10. Procédé selon la revendication 9, **caractérisé en ce que** la préparation initialement appliquée (agent réducteur ou sel d'argent) est utilisée en une concentration au moins deux fois par comparaison avec le deuxième composant et avant application du deuxième composant les fibres de kératine sont tapotées avec un chiffon absorbant ou très brièvement rincées.

11. Procédé selon la revendication 9 ou 10, **caractérisé en ce que** le temps entre l'application de la première préparation et la deuxième préparation va jusqu'à 60 minutes.

12. Procédé selon l'une des revendications 8 à 11, **caractérisé en ce que** la fibre de kératine est chauffée à 50 à 80 °C.

13. Procédé selon l'une des revendications 8 à 12, **caractérisée en ce qu'**avant, pendant ou après avoir donné un effet miroir, la fibre de kératine est colorée de la manière habituelle avec un colorant approprié.

14. Procédé selon la revendication 13, **caractérisé en ce qu'**un colorant non oxydatif est utilisé.

15. Agent bicomposant pour donner un effet miroir à des fibres de kératine, **caractérisé en ce qu'**il est constitué d'une préparation comprenant au moins un composé réducteur exempt de soufre qui est choisi dans le groupe constitué d'acide glyoxylique, glyoxal, glycérylaldéhyde, maltose, acide pyruvique, érythrulose, fructose, lactose, acétoine, acide oxalacétique, acide α-cétoglutarique, dihydroxyacétone, hydroxyméthylglyoxal et 6-aldo-D-fructose et une préparation ammoniacale comprenant au moins un sel d'argent organique ou inorganique.

16. Agent selon la revendication 15, **caractérisée en ce que** le sel d'argent est choisi dans le groupe constitué de nitrate d'argent, chlorure d'argent, sulfate d'argent, carbonate d'argent, hydrogénosulfate d'argent, phosphate d'argent, acétate d'argent et aluns d'argent, et les complexes ammonium ou thiosulfate correspondants de ces sels d'argent.
